# EUROPEAN PATENT SPECIFICATION

(11) **EP 1 615 994 B1**
(45) Date of publication and mention of the grant of the patent: **20.07.2011**
(21) Application number: 04723427.3
(22) Date of filing: 25.03.2004
(51) Int. Cl.: C12N 1/16, B09C 1/10, C02F 3/00

(54) **MICROBIAL MATERIALS FOR DEGRADATION OF OILS AND TOXIC CHEMICALS**
MIKROBIELLE MATERIALIEN ZUM ABBAU VON ÖLEN UND TOXISCHEN CHEMIKALIEN
MATERIAUX MICROBIENS SERVANT A DEGRADER DES HUILES ET DES PRODUITS CHIMIQUES TOXIQUES

(30) Priority: 26.03.2003 KR 2003018913
(43) Date of publication of application: 18.01.2006
(73) Proprietor: Yum, Kyu-Jin, Gyeonggi-do 427-090 (KR)
(72) Inventor: YUM, Kyu-Jin, Kyunggi-Do 427-090 (KR); PARK, Young-Jun, Gwanak-Gu, Seoul 151-050 (KR)
(74) Representative: Svingor, Adam
(86) International application number: PCT/KR2004/000671
(87) International publication number: WO 2004/085626

(56) References cited:
- KR-A- 2000 066 100
- KR-A- 2002 000 444
- US-A- 5 618 727
- LEE PAK-HING ET AL: "Use of solvents to enhance PAH biodegradation of coal tar-contaminated soils" WATER RESEARCH, vol. 35, no. 16, November 2001 (2001-11), pages 3941-3949, XP002370095 ISSN: 0043-1354
- JIMENEZ ILIA Y ET AL: "Solvent-augmented mineralization of pyrene by a Mycobacterium sp" APPLIED AND ENVIRONMENTAL MICROBIOLOGY, vol. 62, no. 7, 1996, pages 2311-2316, XP002370096 ISSN: 0099-2240
- MAY R ET AL: "EX-SITU PROCESS FOR TREATING PAH-CONTAMINATED SOIL WITH PHANEROCHAETE CHRYSOSPORIUM" ENVIRONMENTAL SCIENCE AND TECHNOLOGY, AMERICAN CHEMICAL SOCIETY. EASTON, PA, US, vol. 31, no. 9, September 1997 (1997-09), pages 2626-2633, XP000702296 ISSN: 0013-936X
- GUTIERREZ A ET AL: "The biotechnological control of pitch in paper pulp manufacturing" TRENDS IN BIOTECHNOLOGY, ELSEVIER PUBLICATIONS, CAMBRIDGE, GB, vol. 19, no. 9, 1 September 2001 (2001-09-01), pages 340-348, XP004300201 ISSN: 0167-7799
- ROMERO M.C. ET AL.: 'Isolation and characterization of biarylic structure-degrading yeasts: hydroxylation potential of dibenzofuran' ENVIRON. POLLIT. vol. 118, no. 3, 2002, pages 379 - 382, XP002997548
- LEONE ROBERT ET AL: "Filamentous fungi can degrade aspen steryl esters and waxes", INTERNATIONAL BIODETERIORATION AND BIODEGRADATION, vol. 41, no. 2, March 1998 (1998-03), pages 133-137, ISSN: 0964-8305

## Description

### Technical Field

The present invention relates to a microbial material for degradation of oil and toxic chemicals, and more particularly, to a microbial material which can degrade and treat oils, such as gasoline, naphtha, kerosene or Bunker C oil, and toxic chemicals, such as BTEX (benzene, toluene, ethylbenzene and xylene), which are main oil ingredients.

### Background Art

Major contaminants including, for instance, oils such as gasoline, naphtha, kerosene or Bunker C oil, toxic chemicals such as BTEX, and the like, have been leading to severe environmental contamination of nearby soil and groundwater due to poor degradability and serious toxicity.

Conventional technology for treatment of such contaminants in the soil or groundwater includes physical or chemical treatment, which, however, often has limited applicability because significant running costs are required and the soil ecosystem are severely ruined. One solution that has been proposed in recent years is biological treatment based on biodegradation by indigenous microorganisms in the contaminated soil and groundwater. However, one drawback of the use of biological treatment is that oil-to-oil cohesion or low solubility, which results from lipophilic or hydrophobic properties of oils and toxic chemicals, makes biodegradation by the microorganisms difficult to achieve. Other problems associated with the use of biological treatment include a long process time is required and highly toxic contaminants may adversely affect activity of indigenous microorganisms, considerably lowering the overall treatment efficiency.

### Disclosure of the Invention

To solve the problems of the conventional biological treatment, the present invention provides microbial materials as defined in claim 1.

The microbial nutrient includes degradable saccharide and soybean flour.

The microbial material comprises 1 ~ 10 % by weight of lipophilic powder, 0.1 ~ 1 % by weight of saccharide and 0.0 1 ~ 0.1 % by weight of soybean flour, based on the weight of the microorganism and culture filtrate used.

The microbial material according to the present invention can efficiently, rapidly degrade contaminants that are unreadily degradable, by adding highly lipophilic powder to a composition for preparing the microbial material, so that liophilic or hydrophobic contaminants are naturally adsorbed to the surface of the powder, which increases the effective surface area for biodegradation, consequently increasing a contact area with the microorganism capable of degrading the unreadily degradable contaminants.

Since the contaminants are degraded by the microorganism in a state in which they are adsorbed to the powder surface, the amount of the toxic contaminants induced into the microorganism or adsorbed to the strain is reduced so that the toxicity against to the microorganism is weakened, thereby maintaining a stable biodegrading action.

### Brief description of the drawings

FIG. 1 is a photograph of fluorescence of activated Trichosporon spp. strain colored with acridine orange using a Confocal Laser Microscope with a magnification of 200 times; and
FIG. 2 shows Trichosporon spp. strain in the contaminated soil grown in selective media.

### Best mode for carrying out the Invention

A microbial material according to the present invention will now be described in detail.

The microbial material according to the present invention comprises a mixture comprising a microorganism and culture filtrate capable of degrading oil and toxic chemicals, lipophilic powder and microbial nutrient.

The microorganism is at least one selected from the group consisting of Trichosporon loubieri Y1-A (Of deposit No. KCTC 18079P), Trichosporon cutaneum.

The oils exemplified by gasoline, naphtha, kerosene, Bunker C oil and the like, and the toxic chemicals exemplified by main oil ingredients such as BTEX (benzene, toluene, ethylbenzene, xylene) or chlorophenol compounds, are unreadily degradable and highly toxic, thus causing severe environmental concerns of nearby soil and groundwater.

Useful examples of the microorganism include microorganisms capable of degrading oils and toxic chemicals, preferably at least one selected from the group consisting of Trichosporon spp., Trichosporon cutaneum or a mixed microorganism thereof.

More preferably, the Trichosporon spp. strain is Trichosporon loubieri Y1-A of deposit No. KCTC 18079P newly developed by the present inventors

The lipophilic powder is at least one selected from the group consisting of natural wax, synthetic wax, beeswax and waste candle.

As the particle size of the lipophilic powder such as wax, beeswax or used candle is reduced, the surface area per unit volume of the lipophilic powder increases, making the oils or toxic chemicals easily adsorbed to the powder, so that a contact area with the microorganism is greatly increased, thereby maximizing the efficiency of degrading the oil and toxic chemicals using the microorganism.

In order to enhance a survival rate of the microorganism, the microbial nutrient comprises saccharide, and preferably comprises soybean flour, which is a natural nutrient acting as an electron receptor, in place of oxygen, in an anaerobic condition.

The microbial material comprises 1 - 10 % by weight of lipophilic powder, 0.1 - 1 % by weight of saccharide and 0.01 ~ 0.1 % by weight of soybean flour based on the weight of the microorganism and culture filtrate. Examples of the nutrient include various ingredients beneficial to the microorganism, such as carbon, nitrogen, vitamins or inorganic ions. Glucose or dextrose that is easily utilized by the microorganism without being subjected to biodegradation is preferably used as the source of the carbon, and soybean flour that is a naturally-occurring substance, is preferably used as the source of the nitrogen, vitamins, inorganic ions or electron receptors.

The present invention will now be described in more detail with reference to the following illustrative examples, which are given by way of illustration and are not intended to limit the scope of the present invention.

### EXAMPLE

Trichosporon spp. strain and white-rot fungi were inoculated with 1 mℓ of a spore suspension in 1 ℓ of a nutrient. The nutrient was prepared by adding 24 g of a potato dextrose broth and 10 g of lipophilic powder to 1 ℓ of distilled water and then sterilizing all of them. The spore suspension was prepared by treating a potato agar preculture medium at a low temperature of 4°C or less for 7 days to maximize spore forming capability to 10⁷ spores/mℓ or greater, and then adding 10 mℓ of sterile water thereto. The inoculated microorganisms were cultivated in a continuous aerated incubator to which sterile air of high pressure of 10 psi or higher is applied from a lower portion of a reactor to mix the lipophilic powders and the microorganisms, at a temperature of about 30 to about 35°C for 3 to 5 days, giving a microorganism and culture filtrate. Then, microorganism and culture filtrate, 0.1 ~ 1 % by weight of glucose and 0.01 ~ 0.1 % by weight of soybean flour based on the weight of the microorganism and culture filtrate were mixed, thereby acquiring a desired product, that is, the microbial material according to the present invention.

The microbial material according to the present invention can be advantageously applied for remediation of the soil and groundwater contaminated by chlorine-based organic compounds, agricultural chemicals such as insecticides, herbicides or germicides, gasoline, kerosene, naphtha, Bunker C oil, BTEX (benzene, ethylbezene, toluene, xylene), PAHs (polycyclic aromatic hydrocarbons) and the like.

The oil and other hydrophobic contaminant are not miscible with water in the soil and groundwater but exist in the form of an oil-based contaminant layer. Otherwise, the oil and other hydrophobic contaminant are adsorbed to organic matter in soil, such as humic acid. In the present invention, however, the oil and other hydrophobic contaminant can be adsorbed to surfaces of lipophilic, tiny-sized powder particles contained in the microbial material owing to their high lipophilic and hydrophobic properties. That is, adsorption of the oil and other hydrophobic contaminant to the powder brings about an increased surface area of the contaminants, thereby promoting degradation by selected strains efficiently capable of degrading the contaminants.

The soybean flour, which is a naturally-occurring substance added as a nutrient, acts as a source of nitrogen and vitamin for the selected strains, that is, Trichosporon spp. strain and white-rot fungi. Also, the soybean flour acts as a source of nitrate salts, which are final electron receptors, in the event where oxygen in soil is exhausted during occurrence of the microbial mechanism for degradation, thereby enabling anaerobic degradation by the Trichosporon spp. strain, which is a facultative aerobic microbe. In such a manner, contaminants contained in a clay layer, to which it is quite difficult to feed oxygen, or in groundwater having a very low concentration of dissolved oxygen, can be effectively treated.

### Experimental Example 1

### 1. Preparation of contaminated soil

Diesel oil and naphtha were mixed in the laboratory to prepare 2,400 mℓ of a total petroleum hydrocarbon (TPH) solution with a concentration of 10,000 ppm to then be added to 6,000 mℓ of soil intermittently sterilized at 105°C twice for 2 hours each time, and uniformly mixed, followed by storage in a sealed container for 24 hours at room temperature, thereby finally preparing contaminated soil. Samples consisting of 400 mℓ volumes of soil taken from the thus prepared contaminated soil were placed in 500 mℓ flasks, and 50 mℓ of distilled water was then added to each flask and sealed for adjusting an appropriate water content.

### 2. Treatment of contaminated soil

15 soil samples prepared in the above-described manner were divided into five treatment groups and 3 trials were performed on each treatment group in the following manner. The containers containing 15 samples were sealed and kept at 15 °C for 2 weeks. Thereafter, soil samples were collected from each treatment group and oils was extracted by a soil contamination testing method. Then, gas chromatography was performed using a GC-HP6890 system commercially available from Hewlett-Packard Co., U.S.A. to measure the concentration of TPH. The sealed samples were subjected to intermittent air circulation to construct the same conditions as those of field-site treatments by allowing the containers to be open to be exposed to air for about 1 minute once a day.

### 3. Experiment result

The results of experiments carried out in the above-described manner are summarized in Table 1.

Treatments consist of:
① 0.01 g of wax powder + 0.001 g of glucose + 0.5 mℓ of 100 ppm H₂O₂ + 1 mℓ of Trichosporon spp. microbial culture broth
② 0.01 g of wax powder + 0.001 g of glucose + 0.5 mℓ of 100 ppm H₂O₂
③ 0.001 g of glucose + 0.5 mℓ of 100 ppm H₂O₂ + 1 mℓ of Trichosporon spp. microbial culture broth
④ 0.001 g of glucose + 0.5 mℓ of 100 ppm H₂O₂
⑤ 0.5 mℓ of 100 ppm H₂O₂,
Table 1 shows TPH removal efficiency by treatment.

**Table 1**

| Treatment | ① | ② | ③ | ④ | ⑤ |
|---|---|---|---|---|---|
| TPH removal effiency (%) | 95±2.3 | 51±1.3 | 73±2.5 | 31±1.2 | 23±0.7 |

| | | | | | |
|---|---|---|---|---|---|
| (Average ± standard deviation (3 trials)) | | | | | |

According to the result shown in Table 1, when lipophilic wax powder and microorganisms were used in treatment, the removal efficiency was highest, approximately 22% higher than the efficiency in the case where only the microorganism was used in treatment, which is presumably because biodegradation by the microorganism is promoted by an increased surface area for degradation of the contaminant, the increased surface area occurring when the wax powder treated together with the microorganism infiltrates into the soil and adsorbs the contaminated oil by its inherent liphophilicity.

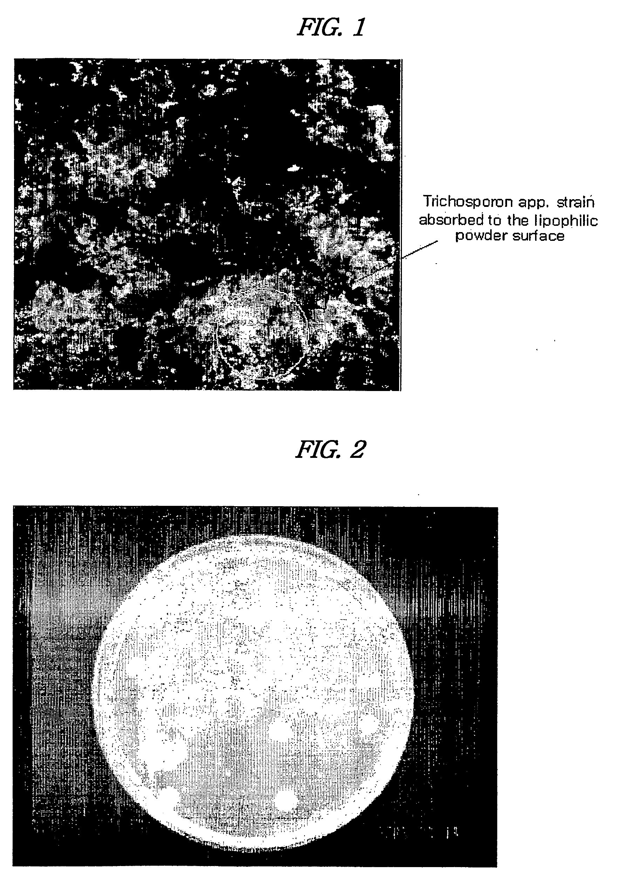

To verify the above-described effects and principles, lipophilic powder was isolated from a soil supernatant of treatment ① in Experimental Example 1, and only surviving cells were selectively colored green using a colorant of Acridine orange. Then, the powder surface was observed while emitting fluorescence using Confocal Laser Microscopy, and the observation result is shown in FIG. 1.

As shown in FIG. 1, the overall surface of lipophilic powder was covered with surviving Trichosporon spp. microorganism cells used together with the powder. This suggest that contaminants adsorbed to the surface of the lipophilic powder are degraded by the Trichosporon spp. strain to be used as a carbon source, allowing the Trichosporon spp. strain cells are densely populated on the surface of the powder (dark green).

### Experimental Example 2

Six samples of soil contaminated by crude oil were collected from six places of different depths in an actual contaminated field-site, sealed, transferred to the laboratory in a low-temperature state, and mixed well.

Treatments consist of:
① 220 g of contaminated soil + 1.5 mℓ of sterilized Trichosporon spp. microbial material
② 220 g of contaminated soil + 1.5 mℓ of Trichosporon spp. microbial material
③ 220 g of contaminated soil + 1.5 mℓ of Trichosporon spp. microbial material cultivated after being mixed with 0.015 g of lipophilic natural wax powder.

The thus treated contaminated soils were sealed and stored at room temperature for 7 days, and the number of surviving Trichosporon spp. microorganisms was counted using selective media for Trichosporon spp. microbial strain. The result is shown in Table 2.

The selective media used were produced from PDA media added with 2 kinds of antibiotics to suppress the growth of indigenous microbes. As shown in FIG. 2, it was confirmed that only Trichosporon spp. strains grew.

Table 2 shows the number of surviving strains of Trichosporon spp. Microorganisms in 1 g of contaminated soil as expressed as colony forming units (cfu)/g.

**Table 2**

| Treatment | ① | ② | ③ |
|---|---|---|---|
| Cfu/soil(g) | 0 | 1.7×10⁴ | 1.4×10⁵ |

| | | | |
|---|---|---|---|
| (Values shown in Table 2 are average values of 3 trials.) | | | |

As apparent from Table 2, when a microbial material and lipophilic powder were used in treatment (③), the concentration of surviving Trichosporon spp. microbial strains was approximately 10 times higher than when only Trichosporon spp. microbial strains were used in treatment (②). Thus, it can be concluded that many Trichosporon spp. microorganism cells are adsorbed to the surface of the lipophilic powder to thus increase resistance to highly toxic contaminants, thereby increasing survival rate.

As described above, the microbial material according to the present invention for degradation of oils has the following advantages.

First, lipophilic ingredients contained in the microbial material increases the efficiency of degradation and the overall period for recovery of the contaminated soil and groundwater can be shortened, thereby reducing the cost for remediation of the contaminated soil and groundwater.

Second, since naturally occurring substances are used as nutrients, the cost for preparation of the microbial material can be reduced.

Third, since the naturally occurring substances containing inorganic ions and various kinds of vitamins are used, the proliferation and activity of selected microorganisms are increased, thereby increasing adaptability to the ecosystem and a survival rate.

Fourth, since the efficiency of degradation of toxic chemicals by the selected microorganism contained in the microbial material and the activity and colony forming units of the indigenous microorganisms are increased, the overall treatment efficiency is enhanced and the oil-contaminated soil and groundwater can be recovered without damaging the soil ecosystem.

Fifth, combination of the present invention treatment with the conventional treatment technologies can synergically increase the efficacy of degradation of soil and groundwater, and environmental problems associated with the conventional treatment can be solved.

### Industrial Applicability

Therefore, the microbial materials for degradation of oil and toxic chemicals according to the present invention can be advantageously used in various applications of the environment industry.

## Claims

1. A microbial material including a mixture comprising a microorganism and culture filtrate capable of degrading oils selected from the group consisting of gasoline, naphtha, kerosene and Bunker C oil, and toxic chemicals selected from the group consisting of benzene, toluene, ethylbenzene and xylene, and further comprising a lipophilic powder and a microbial nutrient, wherein the microorganism is at least one selected from the group consisting of Trichosporon loubieri Y1-A of deposit No. KCTC 18079P and Trichosporon cutaneum, and wherein lipophilic powder is at least one selected from the group consisting of wax, beeswax and waste candle.

2. The microbial material of claim 1, wherein the microbial nutrient includes saccharide and soybean flour.

3. The microbial material of claim 1 or 2, wherein the mixture comprises 1 - 10 % by weight of lipophilic powder, 0.1 ~ 1 % by weight of saccharide and 0.01 ~ 0.1 % by weight of soybean flour based on the weight of the microorganism and culture filtrate used.

## Patentansprüche

1. Mikrobielles Material, enthaltend eine Mischung, umfassend einen Mikroorganismus und Kulturfiltrat, dazu in der Lage, Öle, ausgewählt aus der Gruppe bestehend aus Benzin, schwerem Benzin, Kerosin und Bunker-C-Öl, und toxische Chemikalien, ausgewählt aus der Gruppe bestehend aus Benzen, Toluen, Ethylbenzen und Xylen, abzubauen, und weiter umfassend ein lipophiles Pulver und einen mikrobiellen Nährstoff, wobei der Mikroorganismus mindestens eines ausgewählt aus der Gruppe bestehend aus Trichosporon loubieri Y1-A, Depotnummer KCTC 18079P, und Trichosporon cutaneum ist, und wobei das lipophile Pulver mindestens eines, ausgewählt aus der Gruppe bestehend aus Wachs, Bienenwachs und Kerzenabfall ist.

2. Mikrobielles Material nach Anspruch 1, wobei der mikrobielle Nährstoff Saccharid und Sojabohnenmehl enthält.

3. Mikrobielles Material nach Anspruch 1 oder 2, wobei die Mischung 1 ~ 10 Gew.% lipophiles Pulver, 0,1 ~ 1 Gew.% Saccharid und 0,01 ~ 0,1 Gew.% Sojabohnenmehl basierend auf dem Gewicht des verwendeten Mikroorganismus und Kulurfiltrats umfasst.

## Revendications

1. Matière microbienne comprenant un mélange contenant un micro-organisme et un filtrat de culture capables de dégrader des huiles sélectionnées dans le groupe constitué de l'essence, du naphta, du kérosène et du mazout C, et des produits chimiques toxiques sélectionnés dans le groupe constitué du benzène, du toluène, de l'éthylbenzène et du xylène, et comprenant également une poudre lipophile et un nutriment microbien, dans laquelle le micro-organisme est au moins un micro-organisme sélectionné dans le groupe comprenant la souche Trichosporon loubieri Y1-A déposée sous le numéro KCTC 18079P et l'espèce Trichosporon cutaneum, et dans laquelle la poudre lipophile est au moins une poudre sélectionnée dans le groupe constitué de la cire, de la cire d'abeille et de bougie utilisée.

2. Matière microbienne selon la revendication 1, dans laquelle le nutriment microbien comprend un saccharide et de la farine de fèves de soja.

3. Matière microbienne selon la revendication 1 ou 2, dans laquelle le mélange comprend 1 à environ 10 % en poids de poudre lipophile, 0,1 à environ 1 % en poids de saccharide et 0,01 à environ 0,1 % en poids de farine de fèves de soja sur la base du poids du micro-organisme et du filtrat de culture utilisés.
